(19) Europäisches Patentamt
European Patent Office
Office européen des brevets

(11) **EP 4 407 312 B1**

(12) **EUROPEAN PATENT SPECIFICATION**

(45) Date of publication and mention
of the grant of the patent:
**29.04.2026 Bulletin 2026/18**

(21) Application number: **24152585.6**

(22) Date of filing: **18.01.2024**

(51) International Patent Classification (IPC):
**G01N 33/44** *(2006.01)*     *G01N 30/88* *(2006.01)*

(52) Cooperative Patent Classification (CPC):
**G01N 33/445;** G01N 2030/8854

(54) **METHOD FOR ESTIMATING STATE OF WAX IN RUBBER COMPOUND**

VERFAHREN ZUR SCHÄTZUNG DES WACHSZUSTANDS IN EINER KAUTSCHUKVERBINDUNG

PROCÉDÉ D'ESTIMATION DE L'ÉTAT DE CIRE DANS UN COMPOSÉ DE CAOUTCHOUC

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **27.01.2023 JP 2023010791**

(43) Date of publication of application:
**31.07.2024 Bulletin 2024/31**

(73) Proprietor: **Sumitomo Rubber Industries, Ltd.
Kobe-shi, Hyogo 651-0072 (JP)**

(72) Inventors:
• **IWASAKI, Yuko
Kobe-shi, 651-0072 (JP)**
• **KOMORI, Yoshihiko
Kobe-shi, 651-0072 (JP)**
• **SAKAGUCHI, Yumi
Kobe-shi, 651-0072 (JP)**
• **YAMADA, Hiroaki
Kobe-shi, 651-0072 (JP)**

(74) Representative: **Manitz Finsterwald
Patent- und Rechtsanwaltspartnerschaft mbB
Martin-Greif-Straße 1
80336 München (DE)**

(56) References cited:
CN-A- 107 525 874     JP-A- 2018 189 421
KR-A- 20050 010 406

**Description**

TECHNICAL FIELD

The present invention relates to a method, a program, and a device for estimating a state of wax in a rubber compound.

BACKGROUND ART

KR 2005 0010406 A discloses a method for analyzing wax comprising the steps of: adding a polar solvent to the rubber blend to extract out polar materials; adding a non-polar solvent to the above rubber blend from which polar materials are removed to extract non-polar materials and to provide non-polar extract; forming precipitation in the extract by adding a solvent capable of selectively precipitating wax and then filtering the precipitation to obtain separated wax; and carrying out analysis of the separated wax by using gas chromatography.

JP 2018 189421 A provides an inspection method of a rubber surface including: a transfer step of transferring the wax extracted on the rubber surface to a base material; a dissolution step of immersing the base material in a solvent, so as to dissolve the wax in the solvent; and an inspection step of measuring the carbon number distribution of the wax transferred to the base material.

CN 107 525 874 A discloses a method for determining content of saturates wax and oil in rubber and rubber auxiliaries comprising the steps of providing a solution containing rubber or rubber auxiliary samples or a solution containing rubber or rubber auxiliary sample extracts, adding the solution to a chromatographic column, the solution sequentially passes through a clay adsorbent layer and a silica gel layer in the chromatographic column, the solution passing through the chromatographic column is collected, saturates are obtained after a solvent in the collected solution is removed, an internal standard substance is added to the obtained saturates, the mass percentage of each alkene is calculated through gas chromatography test, the wax content is calculated, the difference between the wax content and the saturate content is the content of oil in the sample, the extracts are obtained after removal of the solvent from extract solution obtained through extraction of rubber and rubber auxiliary samples.

JP 2018-523726T (hereinafter called as "patent literature 1") discloses a wax composition for protecting a rubber product such as a tire from deterioration caused by ozone. The wax composition compounded in the rubber component gradually moves to the surface of the rubber product and forms a wax film. This improves the quality of the rubber product such as ozone resistance.

The above-mentioned wax composition includes hydrocarbons of different chain lengths each having an overall number of carbon atoms in a range of 15 to 110, and has a distribution of hydrocarbons based on the number of carbon atoms per hydrocarbon molecule. Patent literature 1 describes that the analysis of the composition of the wax composition was conducted according to the method described in "Standard Test Method for Analysis of Hydrocarbon Waxes by Gas Chromatography (EWF Method 001/03)" by the European Wax Federation.

SUMMARY OF INVENTION

The deposition amount of the wax composition changes over time. Thus, in order to evaluate the above-mentioned quality of the rubber product, it is important to estimate the current wax content or the like in the rubber product. However, Patent literature 1 only describes the analysis of the composition of the wax composition alone without considering the above-mentioned point.

An object of the present invention is to provide a method, a program, and a device for estimating a state of wax in a rubber compound.

A method for estimating a state of wax in a rubber compound according to a first aspect includes the following:

acquiring a chromatogram of an extracted component, by separating components thereof, in which the wax is extracted from the rubber compound including the wax; and
deriving a distribution of normal hydrocarbons in the wax based on the chromatogram, characterized by the deriving the distribution of the normal hydrocarbons includes:

identifying peaks by carbon atom number for the components separated from the extracted component based on the chromatogram;
among the peaks, removing a specific peak dominated by the component derived from an impurity other than the wax; and
interpolating an area of the removed specific peak based on the areas of the peaks with the carbon atom numbers before and after the carbon atom number corresponding to the specific peak.

The estimation method according to a second aspect is the estimation method according to the first aspect, further including estimating the content of the wax in the rubber compound based on the derived distribution of the normal hydrocarbons.

The estimation method according to another aspect is the estimation method according to the third aspect, in which interpolating the area of the specific peak includes calculating the average value of the areas of the peaks with the carbon atom numbers before and after the carbon atom number.

The estimation method according to another aspect is the estimation method according to any of the first aspect to the fourth aspect, in which the chromatogram is created using a gas chromatograph.

A program for estimating a state of wax in a rubber compound according to the estimation method causes a device to execute the following:

acquiring a chromatogram of an extracted component, by separating components thereof, in which the wax is extracted from the rubber compound including the wax; and
deriving a distribution of normal hydrocarbons in the wax based on the chromatogram.

A device for estimating a state of wax in a rubber compound according the estimation method includes an acquisition portion and a derivation portion. The acquisition portion acquires a chromatogram of a rubber compound including wax by separating components from an extracted component in which the wax is extracted. The derivation portion derives a distribution of normal hydrocarbons in the wax based on the chromatogram.

According to the above-mentioned aspects, it is possible to provide the method, the program, and the device for estimating the state of wax in the rubber compound.

BRIEF DESCRIPTION OF DRAWINGS

FIG. 1 is a graph showing a distribution of normal hydrocarbons created based on peaks influenced by an impurity.
FIG. 2 is graphs showing variations caused by operators correcting the peaks in a chromatogram.
FIG. 3 is a block diagram showing an electrical configuration of an estimation device.
FIG. 4 is a flowchart showing a process of an estimation method.
FIG. 5 is an example of a graph plotting peak areas of the normal hydrocarbons by carbon atom number.
FIG. 6 is a graph of the interpolated peak areas of the normal hydrocarbons.

DESCRIPTION OF EMBODIMENTS

Hereinafter, a method, a program, and a device for estimating a state of wax in a rubber compound according to one embodiment of the present disclosure will be described.

<1. Overview>

A rubber compound described below is a compound in which a rubber component and a non-rubber component other than the rubber component are compounded, and examples thereof include a tire. Examples of the rubber component include natural rubber, butadiene rubber, styrene-butadiene rubber, isoprene rubber, and a mixture thereof. The rubber component may be vulcanized or unvulcanized. Examples of the non-rubber component include an additive, wax, and oil. Examples of the additive include carbon, silica, and zinc oxide.

In the present embodiment, in order to analyze a state of wax in the target rubber compound, data of a chromatogram of an extracted component extracted from the rubber compound is acquired. This extracted component is obtained by extracting at least a part of the non-rubber component from the rubber compound and includes an impurity derived from oil or the like in addition to the wax. A method for extracting the non-rubber component is not particularly limited. However, examples thereof include a method in which the shredded rubber compound is immersed in chlorohexane or cyclohexane for 8 to 24 hours at room temperature, followed by filtration to obtain an extract.

The wax includes an aliphatic hydrocarbon with a molecular weight of about several hundred, and is a mixture of hydrocarbons with about 20 to 50 carbon atoms per molecule. The distribution of these hydrocarbons can be determined, for example, according to the method described in the Standard Test Method for Analysis of Hydrocarbon Waxes by Gas Chromatography (EWF Method 001/03) by the European Wax Federation. According to this method, even a normal/iso ratio, which is the abundance ratio of normal hydrocarbons and iso-hydrocarbons for each carbon atom number, can be determined. In this description, the normal hydrocarbon is a linear hydrocarbon that does not have a branch, and the iso-hydrocarbon is a hydrocarbon that has a branch.

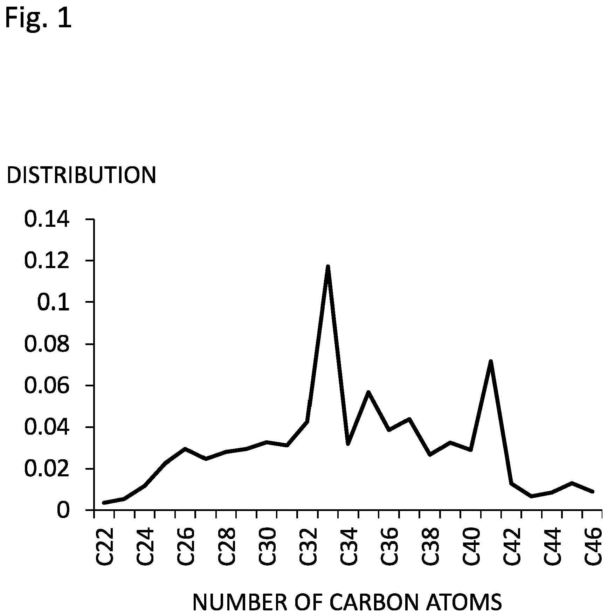

On the other hand, components separated by a gas chromatograph from the extracted component of the rubber compound include a component derived from the wax and a component derived from the impurity. Thus, in the resulting chromatogram, a peak of the component derived from the wax and a peak of the component derived from the impurity overlap with each other. As a result, when peak picking for each carbon atom number is performed using a conventional program, a shape of the graph showing the distribution of hydrocarbons, which is originally smooth, becomes distorted (See FIG. 1. Note that FIG. 1 is a graph showing the distribution of only the normal hydrocarbons). Such a distribution of hydrocarbons is thought to deviate from the actual distribution, and it is impractical to estimate the state of wax based on this distribution.

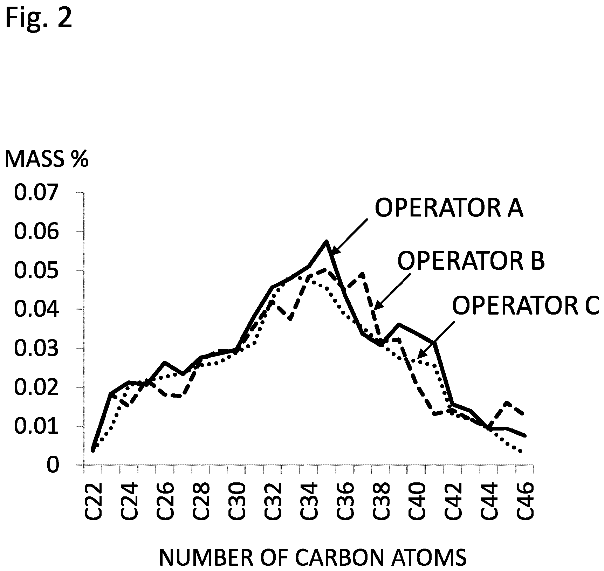

Thus, if the distribution of hydrocarbons in the wax alone is known, there is a method to manually correct the peak (width and top value) of the chromatogram so that the distribution of hydrocarbons in the extracted component is closer to the known distribution. However, the method of manually correcting the peak requires a significant amount of time and causes variations in a degree of correction by operators, causing a decrease in the reliability of the hydrocarbon distribution derived based on the peak area after correction (See FIG. 2). In particular, for the peaks of iso-hydrocarbons whose abundance ratio is relatively small, variations in the peak correction by operators become more pronounced. Further, in the method of comparing the distribution of the extracted component with the known distribution, it is difficult to take into account a change in the distribution of hydrocarbons in the wax over time. Furthermore, this method cannot be applied if the distribution of hydrocarbons in the wax alone is not known.

An estimation device 1 according to the present embodiment makes it possible to execute a more efficient and reliable method for estimating the state of wax in the rubber compound. This estimation method can also be applied to the rubber compound for which the chromatogram of the wax alone is not known. Note that the "state of wax" includes at least one of a distribution of normal hydrocarbons by the carbon atom number in the wax, the total amount of normal hydrocarbons in the wax, and a content ratio and the content of the wax in the rubber compound. Description is provided below.

<2. Estimation device>

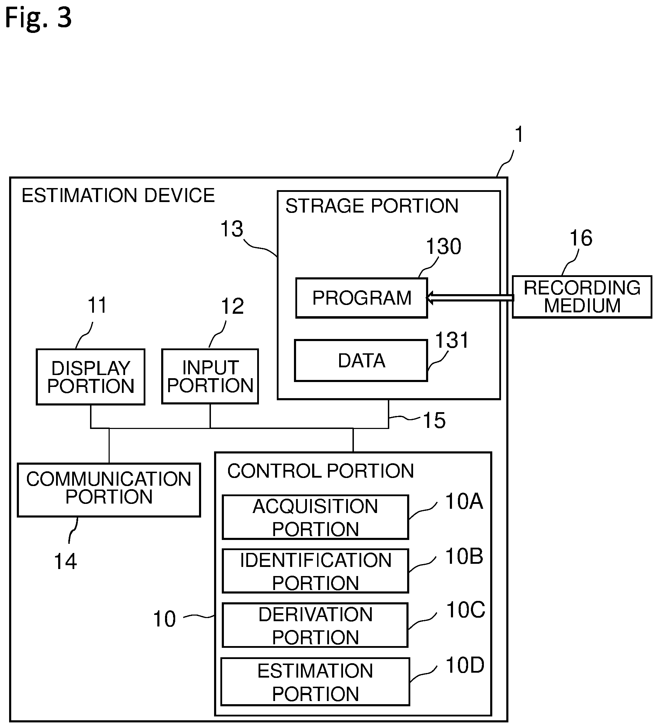

FIG. 3 is a block diagram showing an electrical configuration of the estimation device 1. The estimation device 1 is a general-purpose computer as hardware, and is achieved by, for example, a desktop computer, a laptop computer, a tablet, or a smartphone. The estimation device 1 is produced by, for example, installing an estimation program 130 (hereinafter also simply referred to as "program") into a general-purpose computer from a computer-readable recording medium 16 such as a CD-ROM or USB memory, or via a network.

The estimation device 1 includes a control portion 10, a display portion 11, an input portion 12, a storage portion 13, and a communication portion 14. These portions 10 to 14 are connected to each other via a bus line 15 and can communicate with each other. Further, at least one of the display portion 11, the input portion 12, and the storage portion 13 may be integrated into a main body (casing housing the control portion 10, etc.) of the estimation device 1, or may be externally attached.

The display portion 11 can be configured with a liquid crystal display, an organic EL display, a plasma display, a liquid crystal element, or the like, and displays various pieces of information to a user. The input portion 12 can be configured with a mouse, a keyboard, a touch panel, or the like, and accepts a user operation to the estimation device 1. The communication portion 14 functions as a communication interface that establishes various types of communication connections.

The control portion 10 can be configured with a processor such as a central processing unit (CPU) or a graphics processing unit (GPU), a ROM, a RAM, and the like. The control portion 10 reads and executes the program 130 in the storage portion 13, thereby virtually functioning as an acquisition portion 10A, an identification portion 10B, a derivation portion 10C, and an estimation portion 10D to execute operations described below. The functions of these portions 10A to 10D are achieved by one or more processors.

The storage portion 13 can be configured with a nonvolatile storage device such as a hard disk or a flash memory. The storage portion 13 stores not only the estimation program 130 but also chromatogram data 131 acquired by the acquisition portion 10A, and the like.

<3. Estimation method>

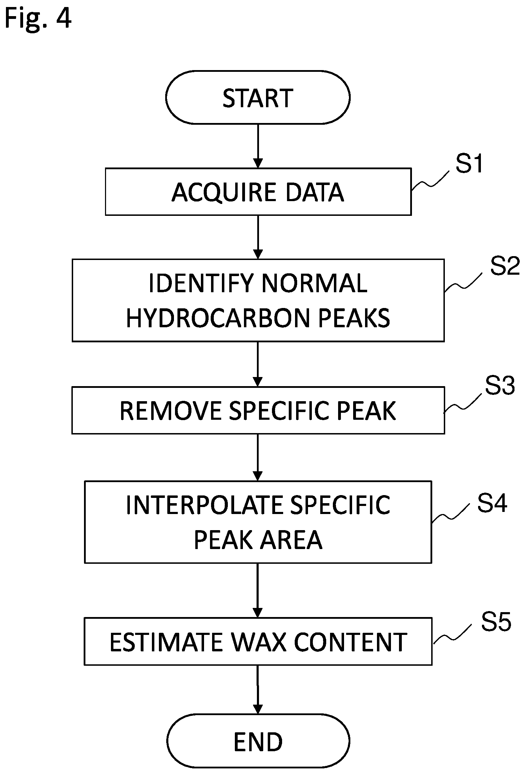

FIG. 4 is a flowchart showing a process of the method for estimating the wax content in the rubber compound, which is executed by the estimation device 1. The processing in FIG. 4 starts, for example, when an instruction to start the estimation process is input from the user via the input portion 12 and accepted by the control portion 10.

First, the acquisition portion 10A acquires a chromatogram of the extracted component to be analyzed and stores the chromatogram as data 131 in the storage portion 13 or RAM (step S1). In the present embodiment, the chromatogram is chart data showing the signal intensity over the retention time, generated by separating components from the above-mentioned extract of the rubber compound using a gas chromatograph and detecting each of the components with a detector. The detector is not particularly limited. However, a flame ionization detector (FID) is preferable. A method for acquiring the chromatogram data is not particularly limited. For example, the data may be read from the gas chromatograph-detector through a wired or wireless data communication, or may be acquired from a recording medium in which the data are stored.

Next, the identification portion 10B reads the data 131 stored in the step S1 and identifies a peak of a normal hydrocarbon with the predetermined number of carbon atoms based on the peaks identified by the chromatogram (step S2). The predetermined number of carbon atoms is not particularly limited. However, it can be, for example, in a range of 20 to 50. More specifically, the identification portion 10B identifies the peaks of the normal hydrocarbons by the carbon atom number based on the retention time known in advance by analysis of their standards. Note that the area of each peak is proportional to the concentration of the normal hydrocarbon with the corresponding carbon atom number in the extract.

Next, the identification portion 10B identifies, as a specific peak, a peak dominated by a component derived from an impurity other than the wax among the peaks identified in the step S2, and removes it from the data 131 (step S3). If the chromatogram of the wax alone is known, the specific peak can be identified by comparing the obtained chromatogram with the known chromatogram of the wax. For example, if the area of the peak of the normal hydrocarbon with some carbon atom number identified in the step S2 deviates from the area of the peak of the normal hydrocarbon with the same carbon atom number identified based on the known chromatogram by a predetermined percentage or more, such a peak can be removed as a specific peak. Alternatively, if a peak top value identified in the step S2 deviates from the corresponding peak top value in the known chromatogram by a predetermined percentage or more, such a peak can be removed as a specific peak.

On the other hand, if the chromatogram of the wax alone is not known or if the distribution of the normal hydrocarbons is thought to have changed significantly, the identification portion 10B identifies the specific peak by comparing the peak for each carbon atom number with the peaks with the preceding and following carbon atom numbers. More specifically, if the peak of interest shows a significantly larger peak top value compared with the preceding and following peaks, if the area of the peak of interest is significantly larger than the areas of the preceding and following peaks, or the like, such a peak can be removed as the specific peak. This is based on the assumption that the content of each normal hydrocarbon in the wax alone does not significantly change by increasing or decreasing one carbon atom number, and the distribution of the normal hydrocarbons by the carbon atom number shows a smooth distribution shape.

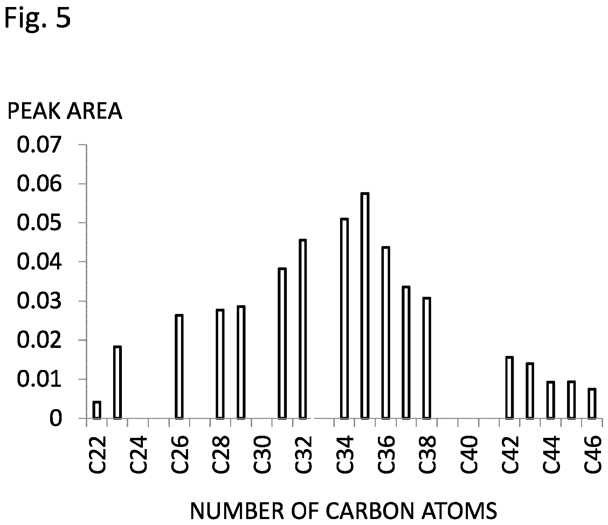

Note that, in the step S3, the peaks with two or more consecutive carbon atom numbers may be removed. As a result, a graph plotting the areas of the peaks of the normal hydrocarbons by the carbon atom number after removing the specific peaks looks to be, for example, a graph shown in FIG. 5.

Subsequently, the derivation portion 10C interpolates the area of the specific peak removed in the step S3 (step S4). This interpolation is performed based on the areas of the peaks with the carbon atom numbers before and after the number of the removed peak. Note that, if the peaks with two or more consecutive carbon atom numbers are removed, interpolation of the two or more peaks is performed based on the areas of the peaks with the carbon atom numbers before and after the numbers in the removed range. The detailed interpolation method is described below.

It is assumed that the peak with the carbon atom number $C_n$ has been removed, and the areas of the peaks with the preceding and following carbon atom numbers $C_{n-1}$ and $C_{n+1}$ are represented by $P_{n-1}$ and $P_{n+1}$, respectively. In this case, the derivation portion 10C calculates the average value, $(P_{n-1}+P_{n+1})/2$, of $P_{n-1}$ and $P_{n+1}$, and takes this value as the area $P_n$ of the peak with the carbon atom number $C_n$. Further, for example, it is assumed that $(k+1)$ peaks with the carbon atom numbers $C_n, C_{n+1}, \ldots, C_{n+k}$ have been removed, and the areas of the peaks with the preceding and following carbon atom numbers $C_{n-1}$ and $C_{n+k+1}$ are represented by $P_{n-1}$ and $P_{n+k+1}$, respectively (where $k \geq 1$). In this case, the derivation portion 10C calculates the areas $P_n, P_{n+1}, \ldots, P_{n+k}$ of the peaks with the carbon atom numbers $C_n, C_{n+1}, \ldots, C_{n+k}$ as follows. Note that d is defined as

$$d=(P_{n+k+1}-P_{n-1})/(k+1).$$

$$P_n=P_{n-1}+d$$

$$P_{n+1}=P_{n-1}+2d$$

$$P_{n+k}=P_{n-1}+(k+1)d$$

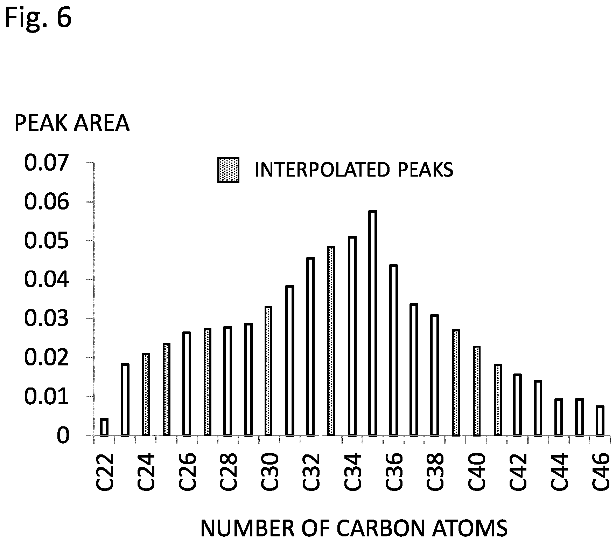

That is, the areas of one or more removed peaks are interpolated such that the average value of the areas including those of the preceding and following peaks is equal to the average value of the areas of the preceding and following peaks alone. Further, the multiple peaks thus removed are interpolated such that the areas of the interpolated peaks increase or decrease by a constant value according to the number of carbon atoms. In other words, interpolation is performed such that the areas of the peaks change in one direction according to the magnitude relationship between the areas of the preceding and following peaks. In this manner, a graph with a smooth shape as shown in FIG. 6 is generated from the graph in FIG. 5, and a distribution of the normal hydrocarbons in the wax is derived in accordance with the above-mentioned assumption.

Referring again to FIG. 4, the estimation portion 10D estimates the content of the wax in the rubber compound based on the distribution of the normal hydrocarbons derived in the step S4 (step S5). The estimation portion 10D converts the sum of the peak areas of the normal hydrocarbons into the content of the normal hydrocarbons in the wax, based on the peak areas of the normal hydrocarbons by the carbon atom number. This conversion can be performed based on the relationship between the peak areas of the normal hydrocarbons and the concentration of the normal hydrocarbons in an adjusted liquid, which is determined in advance by analysis of the normal hydrocarbon standards. Note that, according to studies by the present inventors, if the concentration of the normal hydrocarbons in the adjusted liquid is the same, the peak areas of the normal hydrocarbons are substantially constant regardless of the number of carbon atoms. Thus, if the above-mentioned relationship is determined for one normal hydrocarbon with any carbon atom number, the content of the normal hydrocarbons for all carbon atom numbers can be calculated based on this.

Further, the estimation portion 10D converts the converted normal hydrocarbon content into the wax content. In the present embodiment, this conversion is performed by multiplying the normal hydrocarbon content by a constant. The conversion constant can be determined in advance, for example, based on the assumed abundance ratio of normal hydrocarbons and iso-hydrocarbons in the wax of interest. That is, the converted wax content can be a value corrected for the iso-hydrocarbon content.

Through the above-mentioned procedures, the state of wax including the wax content in the rubber compound and the distribution of the normal hydrocarbons in the wax is estimated. The estimation portion 10D may create a screen that displays the wax content estimated in the step S5, and display this on the display portion 11.

<4. Features>

The estimation method according to the above-mentioned embodiment is executed on the assumption that the distribution of the normal hydrocarbons in the wax alone forms a smooth shape. For this reason, it is relatively easy to identify the specific peak dominantly influenced by an impurity. Further, the value of the removed peak can be interpolated by the simple method. This significantly reduces the work time required to estimate the state of wax, eliminates variations among operators, and improves the reliability in estimating the state of wax.

According to the estimation method according to the above-mentioned embodiment, the state of wax in the rubber compound can be estimated even if the chromatogram of the wax alone included in the rubber compound is unknown or the wax is thought to be deteriorated. Further, if the chromatogram of the wax alone included in the rubber compound is known, the deterioration state of wax in the rubber compound can be estimated by comparing the known distribution of the normal hydrocarbons and the distribution of the normal hydrocarbons derived by the above-mentioned method.

<5. Variations>

One embodiment of the present disclosure has been described above. However, the present disclosure is not limited to the above-mentioned embodiment, and various modifications can be made without departing from the scope of the set of claims.

(1) In the above-mentioned embodiment, the chromatogram of the extracted component of the rubber compound was acquired using the gas chromatograph-flame ionization detector (GC-FID). However, the method for acquiring the chromatogram is not limited to this. For example, a liquid chromatograph may be used instead of the gas chromatograph. Further, as the detector, other detectors such as a mass spectrometer (MS), a thermal conductivity detector (TCD), and a barrier discharge ionization detector (BID) may be used.

(2) The specific peak may be identified by the operator examining the chromatogram of the extracted component. That is, the estimation device 1 may be configured such that the chromatogram acquired in the step S1 is displayed on the display portion 11, the operator having examined the chromatogram specifies the specific peak via the input portion 12, and this input is accepted by the identification portion 10B to remove the specific peak from the chromatogram. The operation of identifying the specific peak has less variations among operators and requires less time than the operation of correcting the specific peak. Thus, even if the specific peak is identified by the operator, the estimation reliability and efficiency can be improved. Alternatively, the specific peak may be identified using a trained machine learning model.

(3) The method for extracting the non-rubber components including the wax from the rubber compound is not limited to the method using cyclohexane, and can be modified as appropriate. Further, the rubber compound is not limited to a tire, and any rubber compound may be used as long as the wax is included.

EXAMPLES

Examples of the present disclosure will be described in detail below. However, the present disclosure is not limited to these examples.

<Experiment 1>

Samples 1 to 3 of rubber compounds each including a rubber component, oil, and wax were prepared. In the samples 1 to 3, the wax content (mass %) was changed to 0.5 times, 1 time, and 2 times, respectively. The total content (mass %) of normal hydrocarbons in this wax was known in advance as shown in Table 1.

[Table 1]

|  | Samples | | |
|---|---|---|---|
|  | 1 | 2 | 3 |
| Normal hydrocarbons | 0.33 | 0.67 | 1.32 |

The samples 1 to 3 were each cut into pieces and immersed in cyclohexane for 24 hours, followed by filtration to prepare an extract in which non-rubber components were extracted. The extracts of the samples 1 to 3 in a required amount were each subjected to a gas chromatograph-flame ionization detector (GC-FID) to obtain a chromatogram. Further, standards for normal hydrocarbons with different carbon atom numbers (C20, C29, C31, and C39) were each dissolved in cyclohexane to a concentration of 0.1 mg/ml to prepare a solution. These solutions were subjected to GC-FID to calculate the peak areas of the standards. The average value of these peak areas was taken as a value corresponding to a normal hydrocarbon concentration of 0.1 mg/ml.

Based on the above-mentioned value corresponding to 0.1 mg/ml and the chromatograms obtained for the samples 1 to 3, the normal hydrocarbon content (mass %) of the samples 1 to 3 was estimated. The estimation method used in Example was the same as the estimation method according to the above-mentioned embodiment. In the estimation method used in Comparative example, an operator identified a specific peak in each obtained chromatogram and corrected the specific peak to be closer to the hydrocarbon peak in the chromatogram of the wax alone.

The analysis conditions using GC-FID were as follows.

Gas chromatograph: GC2010 manufactured by Shimadzu Corp.
Autoinjector: AOC-20is manufactured by Shimadzu Corp.
Injection volume: 1.0 $\mu$L
Sample vaporization chamber temperature: 380°C
Carrier gas: He
Pressure: 100.0 kPa
Column size: film thickness 0.25 $\mu$m, inner diameter 0.25mm, length 15m
Column heating conditions: hold at 160°C for 2 min; raise temperature at 10°C/min; hold at 360°C for 20 min
Detector temperature: 400°C
Makeup gas: He
Makeup flow rate: 30.0 ml/min
Hydrogen flow rate: 40.0 ml/min
Air flow rate: 400.0 ml/min

<Result>

The normal hydrocarbon content estimated for the samples 1 to 3 was as shown in Table 2 below.

[Table 2]

|  | Samples | | |
|---|---|---|---|
|  | 1 | 2 | 3 |
| Normal hydrocarbons | 0.33 | 0.67 | 1.32 |

(continued)

| | Samples | | |
|---|---|---|---|
| | 1 | 2 | 3 |
| Example | 0.39 | 0.67 | 1.22 |
| Comparative example | 2.30 | 2.54 | 3.09 |

As evident from Table 2, the estimation method in Example was capable of estimating the normal hydrocarbon content with higher accuracy than the estimation method in Comparative example. This confirmed the effectiveness of the estimation method according to the above-mentioned embodiment.

LIST OF REFERENCE NUMERALS

1       Estimation device
10A     Acquisition portion
10B     Identification portion
10C     Derivation portion
10D     Estimation portion

**Claims**

1.  A method for estimating a state of wax in a rubber compound, comprising:
    acquiring (S1) a chromatogram of an extracted component, by separating components thereof, in which the wax is extracted from the rubber compound including the wax; and deriving a distribution of normal hydrocarbons in the wax based on the chromatogram, **characterized by** the deriving the distribution of the normal hydrocarbons includes:

        identifying (S2) peaks by carbon atom number for the components separated from the extracted component based on the chromatogram;
        among the peaks, removing (S3) a specific peak dominated by the component derived from an impurity other than the wax; and
        interpolating (S4) an area of the removed specific peak based on the areas of the peaks with the carbon atom numbers before and after the carbon atom number corresponding to the specific peak.

2.  The method for estimating the state of the wax in the rubber compound according to claim 1, further comprising estimating a content of the wax in the rubber compound based on the derived distribution of the normal hydrocarbons.

3.  The method for estimating the state of the wax in the rubber compound according to claim 1, wherein the interpolating the area of the specific peak includes calculating an average value of the areas of the peaks with the carbon atom numbers before and after the carbon atom number.

4.  The method for estimating the state of the wax in the rubber compound according to any one of claims 1 to 3, wherein the chromatogram is created using a gas chromatograph.

5.  A device (1) for estimating a state of wax in a rubber compound according to a method of at least one of claims 1 to 4, comprising:

        an acquisition portion (10A) that acquires a chromatogram of an extracted component, by separating components thereof, in which the wax is extracted from the rubber compound including the wax; and
        a derivation portion (10C) that derives a distribution of normal hydrocarbons in the wax based on the chromatogram by carrying out the steps of:

            identifying peaks by carbon atom number for the components separated from the extracted component based on the chromatogram;among the peaks,
            removing a specific peak dominated by the component derived from an impurity other than the wax; and
            interpolating an area of the removed specific peak based on the areas of the peaks with the carbon atom numbers before and after the carbon atom number corresponding to the specific peak.

6. A program (130) for estimating a state of wax in a rubber compound, causing a device (1) according to claim 5 to execute:

acquiring a chromatogram of an extracted component, by separating components thereof, in which the wax is extracted from the rubber compound including the wax; and
deriving a distribution of normal hydrocarbons in the wax based on the chromatogram.

**Patentansprüche**

1. Verfahren zum Schätzen eines Zustands von Wachs in einer Kautschukzusammensetzung, umfassend:
Erfassen (S1) eines Chromatogramms einer extrahierten Komponente, durch Trennen von Komponenten derselben, worin das Wachs aus der das Wachs enthaltenden Kautschukzusammensetzung extrahiert ist; und Ableiten einer Verteilung normaler Kohlenwasserstoffe in dem Wachs auf Grundlage des Chromatogramms, **dadurch gekennzeichnet, dass** das Ableiten der Verteilung der normalen Kohlenwasserstoffe umfasst:

Identifizieren (S2) von Peaks nach Kohlenstoffatomanzahl für die aus der extrahierten Komponente abgetrennten Komponenten auf Grundlage des Chromatogramms;
aus den Peaks, Entfernen (S3) eines spezifischen Peaks, der durch die Komponente dominiert ist, die aus einer Verunreinigung außer dem Wachs stammt; und
Interpolieren (S4) einer Fläche des entfernten spezifischen Peaks auf Grundlage der Flächen der Peaks mit den Kohlenstoffatomanzahlen vor und nach der Kohlenstoffatomanzahl, die dem spezifischen Peak entspricht.

2. Verfahren zum Schätzen des Zustands des Wachses in der Kautschukzusammensetzung nach Anspruch 1, zudem umfassend das Schätzen eines Gehalts des Wachses in der Kautschukzusammensetzung auf Grundlage der abgeleiteten Verteilung der normalen Kohlenwasserstoffe.

3. Verfahren zum Schätzen des Zustands des Wachses in der Kautschukzusammensetzung nach Anspruch 1, wobei das Interpolieren der Fläche des spezifischen Peaks das Berechnen eines Mittelwerts der Flächen der Peaks mit den Kohlenstoffanzahlen vor und nach der Kohlenstoffanzahl umfasst.

4. Verfahren zum Schätzen des Zustands des Wachses in der Kautschukzusammensetzung nach einem der Ansprüche 1 bis 3, wobei das Chromatogramm unter Verwendung eines Gaschromatographen erzeugt wird.

5. Vorrichtung (1) zum Schätzen eines Zustands von Wachs in einer Kautschukzusammensetzung nach zumindest einem der Ansprüche 1 bis 4, umfassend:

einen Erfassungsabschnitt (10A), der ein Chromatogramm einer extrahierten Komponente erfasst, durch Trennen von Komponenten derselben, worin das Wachs aus der das Wachs enthaltenden Kautschukzusammensetzung extrahiert ist; und
einen Ableitungsabschnitt (10C), der eine Verteilung normaler Kohlenwasserstoffe in dem Wachs auf Grundlage des Chromatogramms ableitet, durch Ausführen der Schritte:

Identifizieren von Peaks nach Kohlenstoffatomanzahl für die aus der extrahierten Komponente abgetrennten Komponenten auf Grundlage des Chromatogramms; aus den Peaks,
Entfernen eines spezifischen Peaks, der durch die Komponente dominiert ist, die aus einer Verunreinigung außer dem Wachs stammt; und
Interpolieren einer Fläche des entfernten spezifischen Peaks auf Grundlage der Flächen der Peaks mit den Kohlenstoffatomanzahlen vor und nach der Kohlenstoffatomanzahl, die dem spezifischen Peak entspricht.

6. Programm (130) zum Schätzen eines Zustands von Wachs in einer Kautschukzusammensetzung, welches eine Vorrichtung (1) nach Anspruch 5 dazu veranlasst, auszuführen:

Erfassen eines Chromatogramms einer extrahierten Komponente, durch Trennen von Komponenten derselben, worin das Wachs aus der das Wachs enthaltenden Kautschukzusammensetzung extrahiert ist; und
Ableiten einer Verteilung normaler Kohlenwasserstoffe in dem Wachs auf Grundlage des Chromatogramms.

**Revendications**

1. Procédé pour estimer l'état d'une cire dans un composé de caoutchouc, comprenant :

   l'acquisition (S1) d'un chromatogramme d'un composant extrait, en séparant des composants de celui-ci, dans lequel la cire est extraite du composé de caoutchouc comprenant la cire ; et l'obtention d'une distribution d'hydrocarbures normaux dans la cire sur la base du chromatogramme,
   **caractérisé en ce que** l'obtention de la distribution des hydrocarbures normaux comprend :

      l'identification (S2) de pics par le nombre d'atomes de carbone pour les composants séparés du composant extrait sur la base du chromatogramme;
      parmi les pics, la suppression (S3) d'un pic spécifique dominé par le composant dérivé d'une impureté autre que la cire ; et
      l'interpolation (S4) d'une zone du pic spécifique supprimé sur la base des zones des pics avec les nombres d'atomes de carbone avant et après le nombre d'atomes de carbone correspondant au pic spécifique.

2. Procédé pour estimer l'état de la cire dans le composé de caoutchouc selon la revendication 1, comprenant en outre l'estimation d'une teneur en cire dans le composé de caoutchouc sur la base de la distribution dérivée des hydrocarbures normaux.

3. Procédé pour estimer l'état de la cire dans le composé de caoutchouc selon la revendication 1, dans lequel l'interpolation de la zone du pic spécifique comporte le calcul d'une valeur moyenne des zones des pics avec les nombres d'atomes de carbone avant et après le nombre d'atomes de carbone.

4. Procédé pour estimer l'état de la cire dans le composé de caoutchouc selon l'une quelconque des revendications 1 à 3, dans lequel le chromatogramme est créé en utilisant un chromatographe en phase gazeuse.

5. Dispositif (1) pour estimer un état de la cire dans un composé en caoutchouc selon un procédé selon au moins une des revendications 1 à 4, comprenant :

   une partie d'acquisition (10A) qui acquiert un chromatogramme d'un composant extrait, en séparant des composés de celui-ci, dans lequel la cire est extraite du composé de caoutchouc comprenant la cire ; et
   une partie de dérivation (IOC) qui obtient une distribution d'hydrocarbures normaux dans la cire sur la base du chromatogramme en réalisant les étapes consistant à :

      identifier des pics par nombre d'atomes de carbone pour les composants séparés du composant extrait sur la base du chromatogramme ; parmi les pics,
      supprimer un pic spécifique dominé par le composant obtenu d'une impureté autre que la cire ; et
      interpoler une zone du pic spécifique enlevé sur la base des zones des pics avec les nombres d'atomes de carbone avant et après le nombre d'atomes de carbone correspondant au pic spécifique.

6. Programme (130) pour estimer l'état d'une cire dans un composé de caoutchouc, amenant un dispositif (1) selon la revendication 5 à exécuter :

   l'acquisition d'un chromatogramme d'un composant extrait, en séparant des composants de celui-ci, dans lequel la cire est extraite du composé de caoutchouc comprenant la cire ; et
   l'obtention d'une distribution d'hydrocarbures normaux dans la cire sur la base du chromatogramme.

Fig. 1

DISTRIBUTION

Fig. 2

# Fig. 3

Fig. 4

START

↓

ACQUIRE DATA — S1

↓

IDENTIFY NORMAL
HYDROCARBON PEAKS — S2

↓

REMOVE SPECIFIC PEAK — S3

↓

INTERPOLATE SPECIFIC
PEAK AREA — S4

↓

ESTIMATE WAX CONTENT — S5

↓

END

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20050010406 A
- JP 2018189421 A
- CN 107525874 A
- JP 2018523726 T